# EUROPEAN PATENT APPLICATION

(11) **EP 4 768 052 A1**
(43) Date of publication of application: **01.07.2026**
(21) Application number: 25227327.1
(22) Date of filing: 29.12.2025
(51) Int. Cl.: A61M 1/34, A61M 1/36, A61M 1/16

(54) **BLOOD PURIFICATION DEVICE AND MEDICAL SYSTEM**

(30) Priority: 27.12.2024 CN 202411982170; 19.12.2025 CN 202511945502
(71) Applicant: Shenzhen Mindray Bio-Medical Electronics Co., Ltd., Shenzhen, Guangdong 518057 (CN)
(72) Inventor: HUANGFU, Yong, Shenzhen, 518057 (CN); HUANG, Jiping, Shenzhen, 518057 (CN); AI, Shiming, Shenzhen, 518057 (CN); PAN, Ruiling, Shenzhen, 518057 (CN)
(74) Representative: KIPA AB

(57) **Abstract**

The application discloses a blood purification device and a medical system, the blood purification device includes a device housing, a blood purifier, a blood pipeline assembly, a power assembly and a blood component analysis assembly. The blood pipeline assembly includes a first blood pipeline connecting a human blood vessel to an inlet of the blood purifier, and a second blood pipeline connecting an outlet of the blood purifier to the human blood vessel. The power assembly includes a blood pump driving blood to flow in the blood pipeline assembly, a first replenishment pump enabling an anticoagulant solution to flow into the first blood pipeline, and/or a second replenishment pump enabling a calcium solution to flow into the second blood pipeline. The blood component analysis assembly is arranged in the device housing, and is configured to detect an acquired blood sample. With the above arrangement, it facilitates corresponding adjustments of anticoagulant drugs and calcium ions in a blood purification circuit.

## Description

### CROSS-REFERENCE TO RELATED APPLICATIONS

This application claims priority to Chinese patent application No. 202411982170.4 filed at China National Intellectual Property Administration (CNIPA) on December 27, 2024 and entitled "BLOOD PURIFICATION DEVICE AND MEDICAL SYSTEM" and Chinese patent application No. 202511945502.6 filed at China National Intellectual Property Administration (CNIPA) on December 19, 2025 and entitled "BLOOD PURIFICATION DEVICE AND MEDICAL SYSTEM".

### TECHNICAL FIELD

The application relates to the technical field of medical devices, and in particular to a blood purification device and a medical system.

### BACKGROUND

A blood purification device usually draws a patient's blood out of the patient's body, removes some metabolic wastes or toxic substances from the blood through certain processes, and then leads the blood back to the body, to achieve a purpose of purifying the blood.

Taking a Continuous Renal Replacement Therapy (CRRT) device as an example, CRRT, as a common treatment technology for extracorporeal blood purification, is a general term of treatment modalities by which moisture and solutes are continuously and slowly removed. After the patient's blood is drawn out of the body, blood coagulation easily occurs when the blood comes into contact with structures of the CRRT device such as pipelines, a dialysis membrane, etc. This makes it necessary to use anticoagulant drugs such as heparin, citric acid or the like during blood purification treatment, to prevent formation of thrombus.

A mechanism of anticoagulant action of the citric acid is: calcium ions are used as an essential factor in a blood coagulation cascade reaction, and citrate achieves an anticoagulant effect by reversibly chelating calcium ions in extracorporeal circulation (ECC). Therefore, the citrate is infused at an arterial end of the ECC, to maintain an ionized calcium level of the ECC at a predetermined parameter (such as 0.25 ~ 0.40 mmol/L) such that local anticoagulation may be achieved effectively; while a calcium-containing solution such as calcium chloride, calcium gluconate or the like is replenished at a venous end of the ECC, or a calcium-containing replacement solution is used at the venous end, to avoid the patient from suffering from hypocalcemia.

However, at present, replenishment of the anticoagulant drugs and calcium ions usually requires acquiring a blood sample or waste liquid manually, and then sending the blood sample or waste liquid to a clinical laboratory, or detecting the blood sample or waste liquid by using a blood gas analyzer or an electrolyte analyzer in the department. It is inconvenient to detect the blood sample or waste liquid, and doctor/nurse cannot obtain a detection result in time. Furthermore, after the doctor/nurse obtains the detection result, the doctor/nurse needs to manually adjust a flow rate/dose of the anticoagulant drugs and/or calcium solutions according to experiences, which is time-consuming, laborious and difficult in operation.

Therefore, how to conveniently adjust the anticoagulant drugs and calcium ions in a blood purification circuit, has become an urgent problem to be solved by those skilled in the art.

### SUMMARY

In view of this, the application provides a blood purification device, to conveniently adjust the anticoagulant drugs and calcium ions in the blood purification circuit. The application further provides a medical system.

In order to achieve the above purpose, the application provides technical solutions as follows.

The application provides a blood purification device, the blood purification device includes a device housing, a blood purifier, a blood pipeline assembly, a power assembly and a blood component analysis assembly.

The blood pipeline assembly includes a first blood pipeline connecting a human blood vessel to an inlet of the blood purifier, and a second blood pipeline connecting an outlet of the blood purifier to the human blood vessel.

The power assembly includes at least one of: a blood pump driving blood to flow in the blood pipeline assembly, a first replenishment pump enabling an anticoagulant solution to flow into the first blood pipeline, or a second replenishment pump enabling a calcium solution to flow into the second blood pipeline.

The blood component analysis assembly is arranged in the device housing, and is configured to detect an acquired blood sample.

Optionally, in the above blood purification device, the blood purification device may further include a controller.

The blood component analysis assembly is further configured to process a detection signal of the blood sample so as to obtain a detection result, and to output the detection result to the controller; or, the blood component analysis assembly is further configured to output a detection signal of the blood sample to the controller, such that the controller processes the detection signal so as to obtain a detection result.

Optionally, in the above blood purification device, the anticoagulant solution may be a citric acid solution, and/or the detection result includes at least a calcium ion concentration in the blood sample.

Optionally, in the above blood purification device, the blood sample may be capable of being acquired by an operator from the first blood pipeline, the second blood pipeline and/or the human blood vessel.

Optionally, in the above blood purification device, the blood sample may be a blood sample in the first blood pipeline or a blood sample in the human blood vessel.

Or, the blood sample is a blood sample in the second blood pipeline.

Optionally, in the above blood purification device, the device housing may be provided with a first surface and a second surface that are opposite to each other and a third surface located between the first surface and the second surface, the first surface is a surface facing the operator.

The blood component analysis assembly has a detection position, the blood sample may be capable of being placed at the detection position from an outside of the device housing, and the detection position is located on the first surface, the second surface or the third surface of the device housing.

Optionally, in the above blood purification device, the device housing may be provided with an accommodation structure, the accommodation structure corresponds to the detection position.

A test card carrying the blood sample, which card is capable of being placed in the accommodation structure, to enable the blood sample to be placed at the detection position.

Optionally, in the above blood purification device, the blood purification device may further include a sampling member, the sampling member is configured to acquire the blood sample from the first blood pipeline and/or the second blood pipeline.

Optionally, in the above blood purification device, the sampling member may be a sampling needle or a sampling pipeline.

The sampling needle or the sampling pipeline is at least partially located in the device housing.

Optionally, in the above blood purification device, the blood sample may be a blood sample in the first blood pipeline.

Or, the blood sample is a blood sample in the second blood pipeline.

Optionally, in the above blood purification device, the controller may be configured to adjust a flow rate of the first replenishment pump and/or the second replenishment pump according to the detection result.

Or, a flow rate of the first replenishment pump and/or the second replenishment pump may be adjusted by an operator according to the detection result.

Optionally, in the above blood purification device, the controller may be configured to provide an adjustment suggestion for the first replenishment pump and/or the second replenishment pump according to the detection result.

Optionally, in the above blood purification device, the blood component analysis assembly may be further configured to detect one or more of a calcium ion concentration, a sodium ion concentration, a potassium ion concentration, a chloride ion concentration and a magnesium ion concentration, in the blood sample.

And/or, the blood component analysis assembly is further configured to detect one or more of a pH value, a pCO2 value, a pO2 value, a Glu value, a Lac value and a Hct value, of the blood sample.

Optionally, in the above blood purification device, the blood purification device may further include an interaction member, the interaction member is communicatively connected to the blood component analysis assembly, and is configured to output a detection result of at least one ion concentration.

Optionally, in the above blood purification device, the interaction member may include a display screen, the display screen is configured to display the detection result, an adjustment suggestion provided according to the detection result, and/or a prompt with corresponding adjustments made according to the detection result.

Or, the interaction member includes a voice part, the voice part is configured to output the detection result, an adjustment suggestion provided according to the detection result, and/or a prompt with corresponding adjustments made according to the detection result.

The application further provides a blood purification device, the blood purification device includes a device housing, a blood purifier, a blood pipeline assembly, a power assembly and a blood component analysis assembly.

The device housing is provided with a connection part.

The blood pipeline assembly includes a first blood pipeline connecting a human blood vessel to an inlet of the blood purifier, and a second blood pipeline connecting an outlet of the blood purifier to the human blood vessel.

The power assembly includes at least one of a blood pump driving blood to flow in the blood pipeline assembly, a first replenishment pump enabling an anticoagulant solution to flow into the first blood pipeline, or a second replenishment pump enabling a calcium solution to flow into the second blood pipeline.

The blood component analysis assembly is provided with a matching part connected to the connection part, and is configured to detect an acquired blood sample.

Optionally, in the above blood purification device, the blood purification device may further include a controller.

The blood component analysis assembly is further configured to process a detection signal of the blood sample so as to obtain a detection result, and to output the detection result to the controller; or, the blood component analysis assembly is further configured to output a detection signal of the blood sample to the controller, such that the controller processes the detection signal so as to obtain a detection result.

Optionally, in the above blood purification device, the anticoagulant solution may be a citric acid solution, and/or the detection result includes at least a detection result of a calcium ion concentration in the blood sample.

Optionally, in the above blood purification device, the controller may be configured to adjust a flow rate of the first replenishment pump and/or the second replenishment pump according to the detection result.

Or, a flow rate of the first replenishment pump and/or the second replenishment pump may be adjusted by an operator according to the detection result.

Optionally, in the above blood purification device, the controller may be configured to provide an adjustment suggestion for the first replenishment pump and/or the second replenishment pump according to the detection result.

Optionally, in the above blood purification device, the connection part may be detachably connected to the matching part.

Optionally, in the above blood purification device, the connection part may be a plug-in box with an opening located on an outer wall of the device housing and recessed toward an interior of the device housing, and the matching part is a housing wall of the blood component analysis assembly.

The blood component analysis assembly is pluggable into the plug-in box.

Optionally, in the above blood purification device, the connection part may be located on an outer wall of the device housing, and the matching part is located on an outer wall of the blood component analysis assembly and is connectable to the connection part.

Optionally, in the above blood purification device, the blood sample may be capable of being acquired by an operator from the first blood pipeline, the second blood pipeline and/or the human blood vessel.

Optionally, in the above blood purification device, the blood sample may be a blood sample in the first blood pipeline or a blood sample in the human blood vessel.

Or, the blood sample is a blood sample in the second blood pipeline.

Optionally, in the above blood purification device, the blood component analysis assembly may have a detection position, and may be provided with an accommodation structure, the accommodation structure corresponds to the detection position.

A test card carrying the blood sample, which card is capable of being placed in the accommodation structure, to enable the blood sample to be placed at the detection position.

Optionally, in the above blood purification device, the blood component analysis assembly may be further configured to detect one or more of a calcium ion concentration, a sodium ion concentration, a potassium ion concentration, a chloride ion concentration and a magnesium ion concentration, in the blood sample.

And/or, the blood component analysis assembly is further configured to detect one or more of a pH value, a pCO2 value, a pO2 value, a Glu value, a Lac value and a Hct value, of the blood sample.

Optionally, in the above blood purification device, the blood purification device may further include an interaction member, the interaction member is communicatively connected to the blood component analysis assembly, and is configured to output a detection result of at least one ion concentration.

Optionally, in the above blood purification device, the interaction member may include a display screen, the display screen is configured to display the detection result, an adjustment suggestion provided according to the detection result, and/or a prompt with corresponding adjustments made according to the detection result.

Or, the interaction member includes a voice part, the voice part is configured to output the detection result, an adjustment suggestion provided according to the detection result, and/or a prompt with corresponding adjustments made according to the detection result.

The application further provides a medical system, the medical system includes at least one blood purification device and a blood gas analysis device.

The at least one blood purification device includes a device housing, a blood purifier, a blood pipeline assembly and a power assembly. The blood pipeline assembly includes a first blood pipeline connecting a human blood vessel to an inlet of the blood purifier, and a second blood pipeline connecting an outlet of the blood purifier to the human blood vessel. The power assembly includes at least one of a blood pump driving blood to flow in the blood pipeline assembly, a first replenishment pump enabling an anticoagulant solution to flow into the first blood pipeline, or a second replenishment pump enabling a calcium solution to flow into the second blood pipeline.

The blood gas analysis device is configured to detect a blood sample, and is capable of being placed relatively independent of the at least one blood purification device and communicatively connected to the at least one blood purification device.

Optionally, in the above medical system, the at least one blood purification device may further include a controller.

The blood gas analysis device is further configured to process a detection signal of the blood sample so as to obtain a detection result, and to output the detection result to the controller; or, the blood gas analysis device is further configured to output a detection signal of the blood sample to the controller, such that the controller processes the detection signal so as to obtain a detection result.

Optionally, in the above medical system, the controller may be configured to adjust a flow rate of the first replenishment pump and/or the second replenishment pump according to the detection result.

Or, a flow rate of the first replenishment pump and/or the second replenishment pump may be adjusted by an operator according to the detection result.

Optionally, in the above medical system, the controller may be configured to provide an adjustment suggestion for the first replenishment pump and/or the second replenishment pump according to the detection result.

Optionally, in the above medical system, the at least one blood purification device and the blood gas analysis device may be arranged in a same medical room.

Optionally, in the above medical system, the at least one blood purification device may include multiple blood purification devices, and the blood gas analysis device is communicatively connected to the multiple blood purification devices.

Or, the at least one blood purification device includes only one blood purification device.

The application further provides a blood purification device, the blood purification device includes a device housing, a blood purifier, a blood pipeline assembly, a power assembly, a controller and a blood component analysis assembly.

The blood pipeline assembly includes a first blood pipeline connecting a human blood vessel to an inlet of the blood purifier, and a second blood pipeline connecting an outlet of the blood purifier to the human blood vessel.

The power assembly includes at least one of a blood pump driving blood to flow in the blood pipeline assembly, a first replenishment pump enabling an anticoagulant solution to flow into the first blood pipeline, or a second replenishment pump enabling a calcium solution to flow into the second blood pipeline.

The blood component analysis assembly is configured to detect an acquired blood sample, and is communicatively connected to the controller.

Optionally, in the above blood purification device, the blood component analysis assembly may be further configured to process a detection signal of the blood sample so as to obtain a detection result, and to output the detection result to the controller; or, the blood component analysis assembly is further configured to output a detection signal of the blood sample to the controller, such that the controller processes the detection signal so as to obtain a detection result.

Optionally, in the above blood purification device, the controller may be configured to adjust a flow rate of the first replenishment pump and/or the second replenishment pump according to the detection result.

Or, a flow rate of the first replenishment pump and/or the second replenishment pump may be adjusted by an operator according to the detection result.

Optionally, in the above blood purification device, the controller may be configured to provide an adjustment suggestion for the first replenishment pump and/or the second replenishment pump according to the detection result.

Optionally, in the above blood purification device, the blood component analysis assembly may be configured to detect one or more of a calcium ion concentration, a sodium ion concentration, a potassium ion concentration, a chloride ion concentration and a magnesium ion concentration, in the blood sample.

And/or, the blood component analysis assembly is configured to detect one or more of a pH value, a pCO2 value, a pO2 value, a Glu value, a Lac value and a Hct value, of the blood sample.

Optionally, in the above blood purification device, the blood sample may be capable of being acquired by an operator from the first blood pipeline, the second blood pipeline and/or the human blood vessel.

Optionally, in the above blood purification device, the blood purification device may further include an interaction member, the interaction member is communicatively connected to the blood component analysis assembly, and is configured to output a detection result of at least one ion concentration.

Optionally, in the above blood purification device, the interaction member may include a display screen, the display screen is configured to display the detection result, an adjustment suggestion provided according to the detection result, and/or a prompt with corresponding adjustments made according to the detection result.

Or, the interaction member includes a voice part, the voice part is configured to output the detection result, an adjustment suggestion provided according to the detection result, and/or a prompt with corresponding adjustments made according to the detection result.

The application further provides a blood purification device, the blood purification device includes a device housing, a blood purifier, a blood pipeline assembly, a power assembly and a blood component analysis assembly.

The blood pipeline assembly includes a first blood pipeline connecting a human blood vessel to an inlet of the blood purifier, and a second blood pipeline connecting an outlet of the blood purifier to the human blood vessel. An anticoagulant solution is capable of flowing into the first blood pipeline, and a calcium solution is capable of flowing into the second blood pipeline.

The power assembly includes a blood pump driving blood to flow in the blood pipeline assembly.

The blood component analysis assembly is arranged in the device housing, and is configured to detect an acquired blood sample.

The application further provides a blood purification device, the blood purification device includes a device housing, a blood purifier, a blood pipeline assembly, a power assembly and a blood component analysis assembly.

The device housing is provided with a connection part.

The blood pipeline assembly includes a first blood pipeline connecting a human blood vessel to an inlet of the blood purifier, and a second blood pipeline connecting an outlet of the blood purifier to the human blood vessel. An anticoagulant solution is capable of flowing into the first blood pipeline, and a calcium solution is capable of flowing into the second blood pipeline.

The power assembly includes a blood pump driving blood to flow in the blood pipeline assembly.

The blood component analysis assembly is provided with a matching part connected to the connection part, and is configured to detect an acquired blood sample.

Optionally, in the above blood purification device, the blood component analysis assembly may be configured to detect one or more of a calcium ion concentration, a sodium ion concentration, a potassium ion concentration, a chloride ion concentration and a magnesium ion concentration, in the blood sample.

And/or, the blood component analysis assembly is configured to detect one or more of a pH value, a pCO2 value, a pO2 value, a Glu value, a Lac value and a Hct value, of the blood sample.

Optionally, in the above blood purification device, the anticoagulant solution may be enabled to flow into the first blood pipeline by an external power device, and/or the calcium solution may be enabled to flow into the second blood pipeline by an external power device.

Optionally, in the above blood purification device, the blood component analysis assembly may be further configured to process a detection signal of the blood sample to obtain a detection result, and to output the detection result to a controller of the blood purification device; or, the blood component analysis assembly is further configured to output a detection signal of the blood sample to the controller of the blood purification device, such that the controller processes the detection signal so as to obtain a detection result.

The controller is configured to send a control signal for adjusting a flow rate of the anticoagulant solution flowing into the first blood pipeline and/or a control signal for adjusting a flow rate of the calcium solution flowing into the second blood pipeline, according to the detection result; or, the controller is configured to provide an adjustment suggestion for the flow rate of the anticoagulant solution flowing into the first blood pipeline and/or the flow rate of the calcium solution flowing into the second blood pipeline, according to the detection result.

The application further provides a medical system, the medical system includes a blood purification device and an auxiliary power device.

The blood purification device uses the blood purification device as described above.

The auxiliary power device includes a first replenishment pump enabling an anticoagulant solution to flow into the first blood pipeline, and/or a second replenishment pump enabling a calcium solution to flow into the second blood pipeline.

Optionally, in the above medical system, the auxiliary power device may be communicatively connected to the blood purification device.

The blood purification device further includes an interaction member, the interaction member includes a display screen, the display screen is configured to display a delivery flow speed of the first replenishment pump and/or the second replenishment pump.

And/or, the blood component analysis assembly is further configured to process a detection signal of the blood sample to obtain a detection result, and to output the detection result to a controller of the blood purification device; or, the blood component analysis assembly is further configured to output a detection signal of the blood sample to the controller of the blood purification device, such that the controller processes the detection signal so as to obtain a detection result. The controller is configured to adjust a flow rate of the first replenishment pump and/or the second replenishment pump according to the detection result; or, the controller is configured to provide an adjustment suggestion for the first replenishment pump and/or the second replenishment pump according to the detection result.

Optionally, in the above medical system, the auxiliary power device may be relatively independent of the blood purification device.

The blood component analysis assembly is further configured to process a detection signal of the blood sample to obtain a detection result, and to output the detection result to a controller of the blood purification device; or, the blood component analysis assembly is further configured to output a detection signal of the blood sample to the controller of the blood purification device, such that the controller processes the detection signal so as to obtain a detection result.

A flow rate of the first replenishment pump and/or the second replenishment pump may be adjusted by an operator according to the detection result.

Optionally, in the above medical system, the auxiliary power device may be provided with a display structure, the display structure is configured to display a delivery flow speed of the first replenishment pump and/or the second replenishment pump.

As may be seen from the above technical solutions, the blood purification device provided in the application may detect the blood sample of the human body through the blood component analysis assembly, such that it is convenient to monitor a blood condition of the human body in time during corresponding operations performed by the blood purification device on the human body, to facilitate adjustment of a corresponding pump in the power assembly to make corresponding adjustments, and facilitate corresponding adjustments of the anticoagulant drugs and calcium ions in the blood purification circuit.

The application further provides a blood purification device, the blood purification device includes a device housing, a blood purifier, a blood pipeline assembly, a power assembly and an electrolyte component analysis assembly.

The blood pipeline assembly includes a first blood pipeline connecting a human blood vessel to an inlet of the blood purifier, and a second blood pipeline connecting an outlet of the blood purifier to the human blood vessel. An anticoagulant solution is capable of flowing into the first blood pipeline, and a calcium solution is capable of flowing into the second blood pipeline.

The power assembly includes a blood pump driving blood to flow in the blood pipeline assembly.

The electrolyte component analysis assembly is arranged in the device housing, and is configured to detect a waste liquid sample generated by a purification operation of acquired blood.

The application further provides a blood purification device, the blood purification device includes a device housing, a blood purifier, a blood pipeline assembly, a power assembly and an electrolyte component analysis assembly.

The device housing is provided with a connection part.

The blood pipeline assembly includes a first blood pipeline connecting a human blood vessel to an inlet of the blood purifier, and a second blood pipeline connecting an outlet of the blood purifier to the human blood vessel. An anticoagulant solution is capable of flowing into the first blood pipeline, and a calcium solution is capable of flowing into the second blood pipeline.

The power assembly includes a blood pump driving blood to flow in the blood pipeline assembly.

The electrolyte component analysis assembly is provided with a matching part connected to the connection part, and is configured to detect a waste liquid sample generated by a purification operation of an acquired blood sample.

Optionally, in the above blood purification device, the electrolyte component analysis assembly may be configured to detect one or more of a calcium ion concentration, a sodium ion concentration, a potassium ion concentration, a chloride ion concentration and a magnesium ion concentration, in the waste liquid sample.

Optionally, in the above blood purification device, the power assembly may further include a first replenishment pump enabling the anticoagulant solution to flow into the first blood pipeline, and/or a second replenishment pump enabling the calcium solution to flow into the second blood pipeline.

Optionally, in the above blood purification device, the anticoagulant solution may be enabled to flow into the first blood pipeline by an external power device, and/or the calcium solution may be enabled to flow into the second blood pipeline by an external power device.

And/or, the blood purification device further includes a sampling member, the sampling member is configured to acquire the waste liquid sample from a waste liquid pipeline or a waste liquid collection device of the blood purification device.

Optionally, in the above blood purification device, the electrolyte component analysis assembly may be configured to process a detection signal of the waste liquid sample to obtain a first detection result, and to output the first detection result to a controller of the blood purification device; or, the electrolyte component analysis assembly is configured to output a detection signal of the waste liquid sample to the controller of the blood purification device, such that the controller processes the detection signal so as to obtain a first detection result.

The controller is configured to issue a control signal for adjusting a flow rate of the calcium solution flowing into the second blood pipeline, according to the first detection result; or, the controller is configured to provide an adjustment suggestion for the flow rate of the calcium solution flowing into the second blood pipeline, according to the first detection result.

Optionally, in the above blood purification device, the electrolyte composition analysis assembly may be further configured to detect an acquired blood sample.

Optionally, in the above blood purification device, the electrolyte component analysis assembly may be configured to process a detection signal of a blood sample so as to obtain a second detection result, and to output the second detection result to a controller of the blood purification device; or, the electrolyte component analysis assembly is configured to output a detection signal of the blood sample to the controller of the blood purification device, such that the controller processes the detection signal so as to obtain a second detection result.

The controller is configured to issue a control signal for adjusting a flow rate of the anticoagulant solution flowing into the first blood pipeline and/or a control signal for adjusting a flow rate of the calcium solution flowing into the second blood pipeline, according to the second detection result; or, the controller is configured to provide an adjustment suggestion for the flow rate of the anticoagulant solution flowing into the first blood pipeline and/or an adjustment suggestion for the flow rate of the calcium solution flowing into the second blood pipeline, according to the second detection result.

The blood purification device provided in the application may detect, through the electrolyte component analysis assembly, the waste liquid sample generated by the purification operation of acquired blood, such that it is convenient to monitor an electrolyte concentration condition of the human body in time during corresponding operations performed by the blood purification device on the human body, to facilitate adjustment of the corresponding pump in the power assembly to make corresponding adjustments, and facilitate corresponding adjustments of the calcium ions in the blood purification circuit.

Since the above blood purification device has the above technical effects, the medical system provided in embodiments of the application has the same technical effects, which will not be described in detail here.

### BRIEF DESCRIPTION OF THE DRAWINGS

In order to explain the embodiments of the application or technical solutions in the related art more clearly, drawings that need to be used in descriptions of the embodiments or the related art will be briefly introduced below. It is apparent that the drawings in the following descriptions are only some embodiments of the application, and other drawings may also be obtained by those of ordinary skill in the art according to these drawings without paying any creative work.
FIG. 1 is a first schematic structural diagram of a blood purification device provided in an embodiment of the application.
FIG. 2 is a second schematic structural diagram of a blood purification device provided in an embodiment of the application.
FIG. 3 is a schematic flowchart of operations of a blood purification device provided in an embodiment of the application.
FIG. 4 is a schematic structural diagram of a medical system provided in an embodiment of the application.

### DETAILED DESCRIPTION

The application discloses a blood purification device, to facilitate adjustments of anticoagulant drugs and calcium ions in a blood purification circuit. The application further provides a medical system.

The technical solutions in the embodiments of the application will be clearly and completely described below with reference to the drawings in the embodiments of the application, and it is apparent that the described embodiments are only a portion of the embodiments of the application, rather than all the embodiments. Based on the embodiments in the application, all other embodiments obtained by those of ordinary skill in the art without paying any creative work fall within the scope of protection of the application.

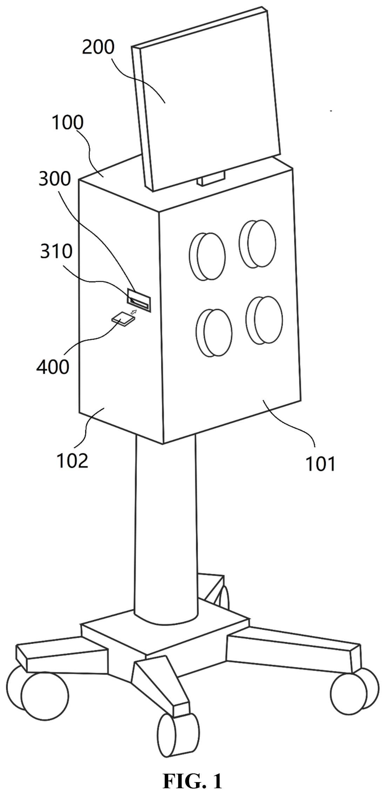

As shown in FIG. 1, an embodiment of the application provides a blood purification device, and the blood purification device includes a device housing 100, a blood purifier, a blood pipeline assembly, a power assembly and a blood component analysis assembly 300.

The device housing 100 may be configured to load some functional members of the blood purification device therein. The functional members may include a portion or all of the above members, i.e., the blood purifier, the blood pipeline assembly, the power assembly and the blood component analysis assembly 300.

Taking a Continuous Renal Replacement Therapy (CRRT) device as an example, the blood purifier may include a filter configured to remove moisture and solutes from blood.

The blood pipeline assembly includes a first blood pipeline connecting a human blood vessel to an inlet of the blood purifier, and a second blood pipeline connecting an outlet of the blood purifier to the human blood vessel. Circulation of blood outside the human body may be achieved through the first blood pipeline and the second blood pipeline. The first blood pipeline is configured to transmit to-be-purified blood delivered by the human blood vessel to the blood purifier, and the second blood pipeline is configured to transmit purified blood delivered by the blood purifier to the human blood vessel.

The power assembly includes a blood pump driving blood to flow in the blood pipeline assembly, a first replenishment pump enabling an anticoagulant solution to flow into the first blood pipeline, and/or a second replenishment pump enabling a calcium solution to flow into the second blood pipeline. The blood pump is used for the first blood pipeline. The blood pump is a power member which enables the blood flowing in the blood pipeline assembly to flow at a certain flow speed, or the flow speed of the blood flowing in the blood pipeline assembly may be controlled by the blood pump. The first replenishment pump may control the anticoagulant solution flowing into the first blood pipeline, including that the first replenishment pump controls whether the anticoagulant solution flows into the first blood pipeline, and controls a flow speed of the anticoagulant solution flowing into the first blood pipeline. The anticoagulant solution is enabled to flow into the first blood pipeline by the first replenishment pump, such that the anticoagulant solution acts on blood in the first blood pipeline, to avoid occurrence of blood coagulation after the blood flows out of the human blood vessel and comes into contact with pipelines of the blood purification device and a filtration structure (such as a dialysis membrane or other structures) of the blood purifier, to ensure an effective purification operation of the blood. The second replenishment pump may control the calcium solution flowing into the second blood pipeline, including that the second replenishment pump controls whether the calcium solution flows into the second blood pipeline, and controls a flow speed of the calcium solution flowing into the second blood pipeline. The calcium solution is enabled to flow into the second blood pipeline by the second replenishment pump, such that the calcium solution acts on blood in the second blood pipeline, to avoid the human body from suffering from hypocalcemia or physical discomfort due to a calcium ion concentration in the blood not falling within a standard range, after the blood flows into the human blood vessel.

A first storage member configured to accommodate the anticoagulant solution and a second storage member configured to accommodate the calcium solution may be detachably arranged on the blood purification device.

The blood component analysis assembly 300 is arranged in the device housing 100, and the blood component analysis assembly is configured to detect an acquired blood sample. An acquisition operation of the blood sample of the human body is implemented by manual sampling or an automatic acquisition device, and the blood sample is placed at a detection position 310 by a manual or mechanical operation, such that the blood component analysis assembly 300 may detect the blood sample.

The blood purification device provided in the embodiment of the application may analyze the blood sample of the human body through the blood component analysis assembly 300, such that it is convenient to monitor a blood condition of the human body in time during corresponding operations performed by the blood purification device on the human body, to facilitate adjustment of the corresponding pump in the power assembly to make corresponding adjustments, and facilitate corresponding adjustments of the anticoagulant drugs and calcium ions in the blood purification circuit.

Furthermore, with adjustment of the calcium ions in the blood purification circuit, it may avoid a situation where the human body suffers from physical discomfort due to a relatively high or low concentration of the calcium ions, and it may play a role of finding and correcting the relatively high or low concentration of the calcium ions in time. With adjustment of the anticoagulant drugs in the blood purification circuit, it may avoid a situation where the blood purification device cannot operate normally due to a relatively high or low dosage of the anticoagulant drugs, and it may ensure an effective implementation of blood purification operation.

The blood purification circuit is a passage for delivering the blood in the blood purification device.

Therefore, in order to facilitate adjustment operations, in some embodiments, the blood purification device further includes a controller. The blood component analysis assembly 300 is further configured to process a detection signal of the blood sample so as to obtain a detection result, and to output the detection result to the controller. That is, after the blood component analysis assembly 300 detects the blood sample and obtains a detection signal, the blood component analysis assembly 300 further processes the detection signal to obtain the detection result (such as a specific concentration value, etc.), the detection result is transmitted by the blood component analysis assembly 300 to the controller, and the controller may perform corresponding processing on the blood purification device according to the detection result. The corresponding processing may include adjustment of the power assembly (the blood pump, the first replenishment pump or the second replenishment pump), start-stop control of the blood purification device or the like, to avoid occurrence of adverse effects due to concentrations of component substances (including the anticoagulant solution and the calcium solution) in the blood failing to reach their standard values. There is a corresponding logic operation to obtain the detection result from the detection signal. Therefore, in the embodiment, the blood component analysis assembly 300 is provided with a member capable of implementing the logic operation individually, such as a circuit board or another member. The member may be relatively independent of a main control board in the blood purification device.

In some other embodiments, the blood purification device further includes a controller. The blood component analysis assembly 300 is further configured to output a detection signal of the blood sample to the controller, such that the controller processes the detection signal so as to obtain a detection result. That is, after the blood component analysis assembly 300 detects the blood sample and obtains a detection signal, the blood component analysis assembly 300 transmits the detection signal to the controller, and the controller processes the detection signal to obtain the detection result (such as a specific concentration value, etc.). The controller may perform corresponding processing on the blood purification device according to the detection result. The corresponding processing may include adjustment of the power assembly (the blood pump, the first replenishment pump or the second replenishment pump), start-stop control of the blood purification device or the like, to avoid occurrence of adverse effects due to concentrations of component substances (including the anticoagulant solution and the calcium solution) in the blood failing to reach their standard values. There is a corresponding logic operation to obtain the detection result from the detection signal, and in the embodiment, the logic operation is implemented by the controller. The controller may be a main control board in the blood purification device, and a unit configured to implement the above logic operation may be integrated into the main control board.

Through multiple systematic reviews included in a Randomized Controlled Trial (RCT), it is pointed out that an extracorporeal circulation (ECC) life of local anticoagulation made by citric acid is better than ECC lives of other anticoagulation modes, and a risk of bleeding in the local anticoagulation made by citric acid is lower. Therefore, preferably, the anticoagulant solution is a citric acid solution. Of course, a component such as heparin or the like may also be used as an anticoagulant component, that is, the anticoagulant solution may also be a heparin solution or another solution, etc.

Furthermore, in an embodiment where citric acid is used as the anticoagulant component, that is, the anticoagulant solution is a citric acid solution, a calcium ion concentration is used as an index for evaluating an anticoagulant effect. Therefore, correspondingly, the detection result includes at least a calcium ion concentration in the blood sample.

Of course, other anticoagulant solutions may also be selected, and the detection result includes at least other detection components capable of evaluating the anticoagulant effect, which will not be described here one by one, and all of which fall within the scope of protection of the application.

In some embodiments, the blood sample may be capable of being acquired by an operator from the first blood pipeline, the second blood pipeline and/or the human blood vessel. The human blood vessel may be a venous blood vessel of the human body. A sampling site of the first blood pipeline may be a section of the pipeline connected between the inlet of the blood purifier and a blood drawing end of the human body. A sampling site of the second blood pipeline may be a section of the pipeline connected between the outlet of the blood purifier and a blood returning end of the human body.

The device housing 100 has a detection position 310 where the acquired blood sample may be placed. During the acquisition operation of the blood sample, the blood sample may be placed at the detection position 310 from an outside of the device housing 100. That is, the detection position 310 is exposed externally, to facilitate the operator to transfer the blood sample to the detection position 310 after completing blood acquisition by a manual or auxiliary device.

The detection position 310 is exposed externally, and the blood sample is a blood sample in the first blood pipeline or a blood sample in the human blood vessel; or, the blood sample is a blood sample in the second blood pipeline. That is, the blood sample may be a first type of blood sample or a second type of blood sample. The first type of blood sample is a sample which is sampled from blood flowing from the human body to the blood purification device, that is, a sample which is sampled from blood drawn out of the body from the human body and required to be processed by the blood purification device. In order to avoid occurrence of blood coagulation due to the blood coming into contact with pipelines transmitting the blood and a purification structure (such as a dialysis membrane), the anticoagulant drugs may be added to the blood after the blood is drawn out of the body. An acquisition point of the first type of blood sample needs to be located at a certain distance before a pipeline section where the anticoagulant drugs are added, to avoid an influence of addition of the anticoagulant drugs on a detection result of the first type of blood sample. The first type of blood sample is a blood sample in the first blood pipeline or a blood sample in the human blood vessel. The second type of blood sample is a sample which is sampled from blood flowing from the blood purification device back to the human body, that is, a sample which is sampled from blood required to flow back to the human body after it is processed by the blood purification device. When the blood flows through the blood purifier, a portion of chelated calcium may be filtered out through the dialysis membrane. Therefore, in order to ensure that a calcium concentration in the blood falls within a standard range to avoid the human body from suffering from hypocalcemia or physical discomfort, it needs to perform an operation of replenishing the calcium solution to the blood before the blood flows back to the human body. An acquisition point of the second type of blood sample needs to be located at a certain distance before a pipeline section where the calcium solution is replenished, to avoid an influence of addition of the calcium solution on a detection result of the second type of blood sample. The second type of blood sample is a blood sample in the second blood pipeline.

As shown in FIG. 1, the device housing 100 is provided with a first surface 101 and a second surface that are opposite to each other and a third surface 102 located between the first surface 101 and the second surface, the first surface 101 is a surface facing the operator. That is, during normal use, the operator operates the blood purification device by facing the first surface 101 of the device housing 100 of the blood purification device. Other reference objects may also be used as an indication of the position of the first surface 101, for example, an orientation of the first surface 101 may be consistent with a display direction of a main display screen 200 of the blood purification device. In a blood purification device of which the main display screen 200 is rotatable relative to the device housing 100, the display direction of the main display screen 200 is the orientation of the first surface 101 when the main display screen 200 is placed in a most commonly used state. Alternatively, multiple operation members of the blood purification device or the like may be provided on the first surface 101.

In some embodiments, the blood component analysis assembly has a detection position 310, the blood sample may be placed at the detection position 310 from an outside of the device housing, and the detection position 310 is located on the first surface 101, the second surface or the third surface 102 of the device housing 100. Preferably, in order to facilitate placing the acquired blood sample at the detection position 310 of the blood component analysis assembly 300, the detection position 310 may be provided on the third surface 102, that is, a side surface of the device housing 100, to avoid interfering with normal operations performed by the operator on the blood purification device (for example, relevant operations of controlling the main display screen 200, observing display data of the main display screen 200, etc.) at the first surface 101 of the blood purification device.

In the embodiment, the device housing is provided with an accommodation structure, the accommodation structure corresponds to the detection position 310. A test card 400 carrying the blood sample may be placed in the accommodation structure, to enable the blood sample to be placed at the detection position 310. The test card 400 may be a member such as a test paper, a glass slide, or an electrode pad, etc. The test card 400 may be used as a disposable member, then after the test card 400 carries the blood sample and is plugged into a plug-in slot to complete detection of the blood sample, the operator may pull out the test card 400, directly treat it as a medical garbage and discard it in a corresponding garbage bin, to vacate the plug-in slot for a next detection operation. The test card 400 may also be used as a recycling member, then after the test card 400 carries the blood sample and is plugged into a plug-in slot to complete detection of the blood sample, the operator may pull out the test card 400 and perform corresponding cleaning and disinfection operations on the test card, to facilitate using the test card next time.

The accommodation structure may have a function of limiting or loading the test card 400. Specifically, the accommodation structure may be a plug-in slot, or a cavity with an on/off door, etc.

In an embodiment where the accommodation structure is a plug-in slot, the test card 400 is pluggably arranged in the plug-in slot, and in a state where the test card 400 is inserted into the plug-in slot, the blood sample carried by the test card 400 is arranged corresponding to the detection position 310.

In an embodiment where the accommodation structure is a cavity with an on/off door, the cavity may be located in the device housing, and a function of shielding the cavity is achieved by the on/off door. The test card 400 may be placed in the cavity by opening the on/off door, and the cavity may be closed by closing the on/off door. In a state where the test card 400 is placed in the cavity, the blood sample carried by the test card 400 is arranged corresponding to the detection position 310.

Of course, the accommodation structure may also be set as other structures.

Since the above accommodation structure (the plug-in slot or the cavity with an on/off door) enables the blood sample carried by the test card 400 to be located in the device housing, it avoids a situation where an detection terminal of the blood component analysis assembly 300 for detecting the blood sample is exposed externally, and it effectively improves a protective effect of the blood component analysis assembly 300. In the embodiment, the blood component analysis assembly 300 may be integrated inside the device housing 100. Of course, a housing of the blood component analysis assembly 300 may also be fixed relative to the device housing 100.

The detection position 310 may also be set as with other structures, such as a carrying platform, such that the blood sample or the test card 400 carrying the blood sample may be placed on the carrying platform directly, to facilitate the detection terminal of the blood component analysis assembly 300 to detect the blood sample placed on the carrying platform directly or indirectly. In the embodiment, the blood sample carried by the test card 400 may be located outside the device housing, or may be located in the device housing.

In some other embodiments, during the acquisition operation of the blood sample, the blood sample may be placed at the detection position 310 from inside of the device housing 100. That is, the detection position 310 is built into the device housing 100, to reduce a probability of contamination of the blood sample during detection. The detection position 310 is enclosed by the device housing 100 to be isolated from an outside of the blood purification device.

The blood purification device further includes a sampling member, the sampling member is configured to acquire the blood sample from the first blood pipeline and/or the second blood pipeline. The sampling member of the blood purification device acquires the blood sample from the first blood pipeline. A sampling site of the first blood pipeline may be located in a pipeline section of the first blood pipeline outside the device housing 100, that is, the blood sample is acquired from the pipeline section of the first blood pipeline outside the device housing 100. Similarly, the sampling member of the blood purification device acquires the blood sample from the second blood pipeline. A sampling site of the second blood pipeline may be located in a pipeline section of the second blood pipeline outside the device housing 100, that is, the blood sample is acquired from the pipeline section of the second blood pipeline outside the device housing 100. There may be one or multiple sampling members for sampling from the first blood pipeline and the second blood pipeline. One sampling member may be used to complete sampling from sampling sites of the first blood pipeline and the second blood pipeline by moving position of this sampling member; or, multiple sampling members may be provided to sample from sampling sites of the first blood pipeline and the second blood pipeline respectively, that is, the sampling member which samples from the sampling site of the first blood pipeline is different from the sampling member which samples from the sampling site of the second blood pipeline.

The sampling member is a sampling needle or a sampling pipeline. The sampling needle or the sampling pipeline is at least partially located in the device housing. In an embodiment where the sampling member is a sampling needle, the sampling needle may pass through sampling sites of the first blood pipeline and the second blood pipeline and perform a sampling operation of bloods in the blood pipelines. In an embodiment where the sampling member is a sampling pipeline, the sampling pipeline may be used as a diversion pipeline of each of the first blood pipeline and the second blood pipeline, to divert bloods in the blood pipelines to complete sampling, to facilitate the blood sample to reach the detection position 310 with guidance of the sampling pipeline, thereby detecting the blood sample.

Of course, the sampling member may use other structures, as long as they may complete sampling of bloods in the first blood pipeline and the second blood pipeline and transferring the sampled bloods to the detection position 310 to detect the sampled bloods, which are not specifically limited here, and all of which fall within the scope of protection of the application.

In the embodiment, the blood sample is a blood sample in the first blood pipeline; or, the blood sample is a blood sample in the second blood pipeline. That is, the blood sample may be a first type of blood sample or a second type of blood sample. The first type of blood sample may be a sample which is sampled from blood flowing from the human body to the blood purification device, that is, a sample which is sampled from blood drawn out of the body from the human body and required to be processed by the blood purification device. In order to avoid occurrence of blood coagulation due to the blood coming into contact with pipelines transmitting the blood and a purification structure (such as a dialysis membrane), the anticoagulant drugs may be added to the blood after the blood is drawn out of the body. An acquisition point of the first type of blood sample needs to be located at a certain distance before a pipeline section where the anticoagulant drugs are added, to avoid an influence of addition of the anticoagulant drugs on a detection result of the first type of blood sample. The first type of blood sample is a blood sample in the first blood pipeline. The second type of blood sample may be a sample which is sampled from blood flowing from the blood purification device back to the human body, that is, a sample which is sampled from blood required to flow back to the human body after it is processed by the blood purification device. When the blood flows through the blood purifier, a portion of chelated calcium may be filtered out through the dialysis membrane. Therefore, in order to ensure that a calcium concentration in the blood falls within a standard range to avoid the human body from suffering from hypocalcemia or physical discomfort, it needs to perform an operation of replenishing the calcium solution to the blood before the blood flows back to the human body. An acquisition point of the second type of blood sample needs to be located at a certain distance before a pipeline section where the calcium solution is replenished, to avoid an influence of addition of the calcium solution on a detection result of the second type of blood sample. The second type of blood sample is a blood sample in the second blood pipeline.

Adjustment magnitudes of flow rates of the anticoagulant solution and the calcium solution are related to concentrations of the anticoagulant solution and the calcium solution used respectively, and doctor/nurse needs to manually calculate corresponding adjustment results of their flow speeds. For example, "adjustment table of local anticoagulation made by sodium citrate-calcium" may be posted on the CRRT device. The doctor/nurse may look up the table based on monitoring the calcium ion concentration in blood after the filter and in the patient's body, to determine how to adjust flow speeds of sodium citrate and the calcium solution. However, the flow speed of sodium citrate is related to the flow speed of the blood, and the above form of table is only applicable to a specific flow speed of the blood (such as 150 ml/min). Furthermore, the above table defines solutions in specific concentrations (for example, a citrate salt commonly used clinically includes a high concentration of 4% citric acid in 136 mmol/L, a low concentration of citric acid (such as Baxter Regiocit) in 18 mmol/L; a calcium solution commonly used clinically includes 10% calcium gluconate and 5% calcium chloride). If the solutions are replaced by other concentrations of sodium citrate or other calcium solutions, the above table cannot be used to guide flow speed adjustment. Furthermore, the doctor/nurse needs to look up the table and perform calculation manually, and there is a high probability of occurrence of errors.

Therefore, in order to facilitate the flow speed adjustment and reduce the error probability, in the blood purification device according to the embodiment of the application, the controller is configured to adjust a flow rate of the first replenishment pump and/or the second replenishment pump according to the detection result. That is, the controller may perform corresponding processing on the blood purification device according to the detection result, the corresponding processing includes adjustment of the flow rate of the first replenishment pump and adjustment of the flow rate of the second replenishment pump. With adjustment of the flow rate of the first replenishment pump, it is convenient to control whether the anticoagulant solution flows into the first blood pipeline and adjust a flow rate of the anticoagulant solution flowing into the first blood pipeline, thereby adjusting a concentration of the anticoagulant component in the blood. With adjustment of the flow rate of the second replenishment pump, it is convenient to control whether the calcium solution flows into the second blood pipeline and adjust a flow rate of the calcium solution flowing into the second blood pipeline, thereby adjusting a concentration of the calcium ions in the blood. The controller may adjust the flow rate of the first replenishment pump and/or the second replenishment pump according to the detection result, to implement automatic adjustment.

Or, the detection result may be directly obtained by an operator, and a flow rate of the first replenishment pump and/or the second replenishment pump may be adjusted by the operator according to the detection result. The detection result may include a specific concentration value or the like, or may include an indication that a corresponding component is relatively high or low (for example, an information indication such as "a (venous) free calcium concentration after the filter: 0.45 mmol/L (relatively high)", etc.) or the like, to facilitate the operator to manually adjust the flow rate of the first replenishment pump and/or the second replenishment pump according to the detection result.

Or, the controller may provide an adjustment suggestion for the first replenishment pump and/or the second replenishment pump according to the detection result. The adjustment suggestion may include specific adjustment information for the first replenishment pump and/or the second replenishment pump. For example, the adjustment suggestion has an information indication such as "It is suggested to increase a flow peed of 4% sodium citrate by 5 ml/h" or the like, to facilitate the operator to obtain the adjustment suggestion and determine whether adjustment is required according to an actual situation. There are individual differences in different organisms, therefore by obtaining the above adjustment suggestion, it facilitates the operator to determine whether adjustment is performed in combination with an individual difference of an operated object (patient), to perform an increasing or decreasing adjustment according to the adjustment information, etc.

Specifically, the blood component analysis assembly 300 may be further configured to detect one or more of a calcium ion concentration, a sodium ion concentration, a potassium ion concentration, a chloride ion concentration and a magnesium ion concentration, in the blood sample. Taking the sodium ion concentration as an example, during treatment of the patient with the blood purification device, for the patient suffering from hypernatremia or hyponatremia, the sodium ion concentration in the blood sample is detected by the blood component analysis assembly 300, to facilitate monitoring the sodium ion concentration in the blood, and facilitate finding and correcting the above various diseases in time. Similarly, during treatment of the patient with the blood purification device, for the patient suffering from hyperkalemia, hypokalemia or other abnormal electrolyte levels, it also needs to monitor corresponding electrolyte concentrations in the blood at a regular time.

In a process of monitoring the above ion concentrations, the blood purification device may also provide a warning reminder according to the detection result, to remind the doctor/nurse to find the patient's symptom in time and make corresponding processing. For example, the blood purification device may provide a reminder message such as "Sodium ion concentration is too high, please process it!", etc. Or, concentrations of various ions monitored at different time points may be presented in a form of trend graph, to facilitate the doctor/nurse to observe and review status of the patient throughout the treatment process.

Furthermore, the blood component analysis assembly 300 may also be configured to detect one or more of a pH (hydrogen ion concentration) value, a pCO2 (Partial Pressure of Carbon Dioxide) value, a pO2 (partial pressure of oxygen) value, a Glu (glucose) value, a Lac (Lactate) value and a Hct (Hematocrit) value, of the blood sample. Through corresponding detection operations, it facilitates the operator to perform corresponding processing according to the detection result.

In order to facilitate the operator to obtain the detection result, the blood purification device further includes an interaction member, the interaction member is communicatively connected to the blood component analysis assembly 300, and may output a detection result of at least one ion concentration. The interaction member may implement interaction between the detection result of the blood component analysis assembly 300 and the operator, to facilitate the operator to obtain, through the interaction member, the detection result of the blood component analysis assembly 300 for at least one ion concentration output by the interaction member.

Specifically, in some embodiments, the interaction member includes a display screen, the display screen is configured to display the detection result, an adjustment suggestion provided according to the detection result, and/or a prompt with corresponding adjustments made according to the detection result. The display screen may be relatively independent of the main display screen 200 of the blood purification device, or the display screen may be integrated with the main display screen 200 of the blood purification device.

In other embodiments, the interaction member includes a voice part, the voice part is configured to output the detection result, an adjustment suggestion provided according to the detection result, and/or a prompt with corresponding adjustments made according to the detection result. The voice part may be an audio system, or a signal transmission member connected to an external audio system, etc. A prompt voice broadcasted by the voice part may be set according to requirements, to facilitate outputting broadcast information in languages of different countries.

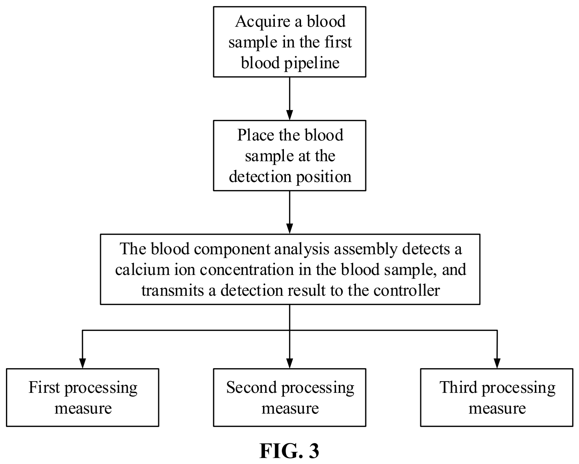

As shown in FIG. 1 and FIG. 3, a specific operation flow of the blood purification device provided in the embodiment of the application is as follows.

A blood sample in the first blood pipeline is acquired. In this process, the blood sample may be acquired manually or automatically.

The blood sample is placed at the detection position 310. The blood sample may be directly placed at the detection position 310, or may be indirectly placed at the detection position 310 through the test card 400 carrying the blood sample.

The blood component analysis assembly detects a calcium ion concentration in the blood sample, and transmits a detection result to the controller.

The controller performs corresponding processing according to the detection result, the corresponding processing includes a first processing measure, a second processing measure and a third processing measure.

The first processing measure may be that the controller adjusts a flow speed of the first replenishment pump or the second replenishment pump according to the detection result, to adjust a flow speed of the citric acid solution or the calcium solution flowing into the first replenishment pump or the second blood pipeline (for example, when it is detected according to the detection result of the blood sample in the first blood pipeline that the calcium ion concentration is greater than 1.2 mmol/L and the calcium solution flowing into the second blood pipeline is 10% calcium gluconate, the flow speed of the second replenishment pump decreases by 3 ml/h).

The second processing measure may be that the controller is configured to provide a suggestion for adjusting the flow speed of the first replenishment pump or the second replenishment pump according to the detection result, and the blood purification device adjusts the flow speed of the first replenishment pump or the second replenishment pump after a user confirms the suggestion, to adjust the flow speed of the citric acid solution or the calcium solution flowing into the first replenishment pump or the second blood pipeline (for example, the display screen of the interaction member displays or the voice part broadcasts that "the calcium ion concentration of the blood sample in the first blood pipeline is greater than 1.2 mmol/L, and the calcium solution flowing into the second blood pipeline is 10% calcium gluconate, then it is suggested decreasing the flow speed of the second replenishment pump by 3 ml/h. Please confirm whether changes are made". After the user confirms it, the flow speed of the second replenishment pump is changed.)

The third processing measure may be that the controller is is configured to provide a suggestion for adjusting the flow speed of the first replenishment pump or the second replenishment pump according to the detection result, to facilitate the user to determine by himself whether the first replenishment pump or the second replenishment pump is adjusted (for example, the display screen of the interaction member displays or the voice part broadcasts that "the calcium ion concentration of the blood sample in the first blood pipeline is greater than 1.2 mmol/L", which is intended to remind the operator to determine by himself whether the second replenishment pump is adjusted, according to the detection result).

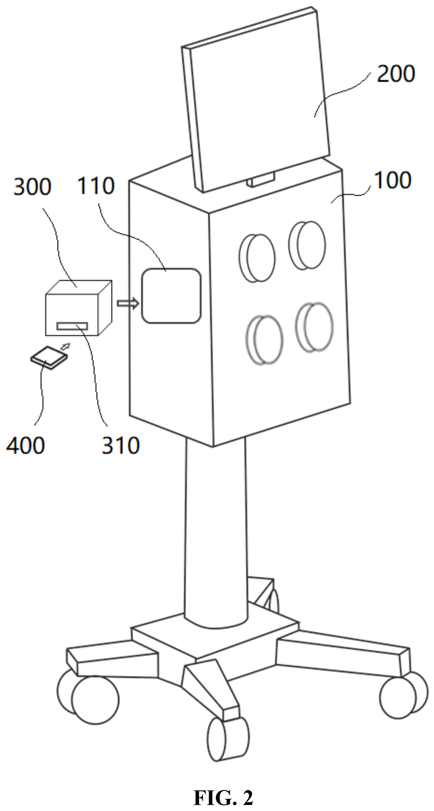

As shown in FIG. 2, an embodiment of the application further provides a blood purification device, and the blood purification device includes a device housing 100, a blood purifier, a blood pipeline assembly, a power assembly and a blood component analysis assembly 300.

The device housing 100 may be configured to load some functional members of the blood purification device therein. The functional members may include a portion or all of the above members, i.e., the blood purifier, the blood pipeline assembly and the power assembly.

Taking a CRRT device as an example, the blood purifier may include a filter configured to remove moisture and solutes from blood.

The blood pipeline assembly includes a first blood pipeline connecting a human blood vessel to an inlet of the blood purifier, and a second blood pipeline connecting an outlet of the blood purifier to the human blood vessel. Circulation of blood outside the human body may be achieved through the first blood pipeline and the second blood pipeline. The first blood pipeline is configured to transmit to-be-purified blood delivered by the human blood vessel to the blood purifier, and the second blood pipeline is configured to transmit purified blood delivered by the blood purifier to the human blood vessel.

The power assembly includes a blood pump driving blood to flow in the blood pipeline assembly, a first replenishment pump enabling an anticoagulant solution to flow into the first blood pipeline, and/or a second replenishment pump enabling a calcium solution to flow into the second blood pipeline. Similarly, the blood pump is used for the first blood pipeline. The blood pump is a power member which enables the blood flowing in the blood pipeline assembly to flow at a certain flow speed, or the flow speed of the blood flowing in the blood pipeline assembly may be controlled by the blood pump. The first replenishment pump may control the anticoagulant solution flowing into the first blood pipeline, including that the first replenishment pump controls whether the anticoagulant solution flows into the first blood pipeline, and controls a flow speed of the anticoagulant solution flowing into the first blood pipeline. The anticoagulant solution is enabled to flow into the first blood pipeline by the first replenishment pump, such that the anticoagulant solution acts on blood in the first blood pipeline, to avoid occurrence of blood coagulation after the blood flows out of the human blood vessel and comes into contact with pipelines of the blood purification device and a filtration structure (such as a dialysis membrane or other structures) of the blood purifier, to ensure an effective purification operation of the blood. The second replenishment pump may control the calcium solution flowing into the second blood pipeline, including that the second replenishment pump controls whether the calcium solution flows into the second blood pipeline, and controls a flow speed of the calcium solution flowing into the second blood pipeline. The calcium solution is enabled to flow into the second blood pipeline by the second replenishment pump, such that the calcium solution acts on blood in the second blood pipeline, to avoid the human body from suffering from hypocalcemia or physical discomfort due to the calcium ion concentration in the blood not falling within a standard range, after the blood flows into the human blood vessel.

A first storage member configured to accommodate the anticoagulant solution and a second storage member configured to accommodate the calcium solution may be detachably arranged on the blood purification device.

In the embodiment, the device housing 100 is provided with a connection part, and the blood component analysis assembly 300 is provided with a matching part connected to the connection part. The blood component analysis assembly is configured to detect an acquired blood sample.

The blood component analysis assembly 300 may have a detection position 310, and the blood component analysis assembly 300 may be connected to the connection part of the device housing 100 through the matching part, to implement a physical connection between the blood component analysis assembly 300 and the device housing 100. In a state where the connection part is connected to the matching part, the blood component analysis assembly 300 has a detection position 310 exposed externally, the acquired blood sample may be placed at the detection position 310, and the blood component analysis assembly 300 may detect the blood sample placed at the detection position 310.

Similarly, the blood purification device provided in the embodiment of the application facilitates corresponding adjustments of the anticoagulant drugs and calcium ions in the blood purification circuit.

Furthermore, with adjustment of the calcium ions in the blood purification circuit, it may avoid a situation where the human body suffers from physical discomfort due to a relatively high or low concentration of the calcium ions, and it may play a role of finding and correcting the relatively high or low concentration of the calcium ions in time. With adjustment of the anticoagulant drugs in the blood purification circuit, it may avoid a situation where the blood purification device cannot operate normally due to a relatively high or low dosage of the anticoagulant drugs, and it may ensure an effective implementation of blood purification operation.

The blood purification device in the embodiment further includes a controller. The blood component analysis assembly 300 is further configured to process a detection signal of the blood sample so as to obtain a detection result, and to output the detection result to the controller; or, the blood component analysis assembly 300 is further configured to output a detection signal of the blood sample to the controller, such that the controller processes the detection signal so as to obtain a detection result.

Furthermore, as described in the previous blood purification device, the anticoagulant solution is a citric acid solution, and/or the detection result includes at least a detection result of a calcium ion concentration in the blood sample. Of course, a component such as heparin or the like may also be used as an anticoagulant component, that is, the anticoagulant solution may also be a heparin solution or another solution, etc.

Control modes of the controller are the same as or similar to control modes of the controller described in the previous blood purification device, which will not be described in detail here.

In order to improve flexibility of using the blood purification device, the connection part is detachably connected to the matching part. That is, with relative detachment between the connection part and the matching part, the blood component analysis assembly 300 may be made independent of other structures of the blood purification device, to facilitate repair and maintenance operations of the blood component analysis assembly 300, for example, charging or replacing the blood component analysis assembly 300, etc. The blood component analysis assembly 300 may be provided with a power supply battery, and the power supply battery may store an amount of electricity when the blood component analysis assembly 300 is charged, such that the power supply battery may supply power to the operation of detecting the blood sample by the blood component analysis assembly 300. That is, the blood component analysis assembly 300 may be supplied with power by the power supply battery, in a state where an external power supply is not connected thereto (for example, the blood component analysis assembly 300 is not electrically connected to a power supply structure of the blood purification device).

As shown in FIG. 2, in a specific embodiment, the connection part is a plug-in box 110 with an opening located on an outer wall of the device housing 100 and recessed toward an interior of the device housing 100, and the matching part is a housing wall of the blood component analysis assembly 300; the blood component analysis assembly 300 may be plugged into the plug-in box 110. That is, the blood component analysis assembly 300 may be used as a plug-in member to plug into and cooperate with the plug-in box 110 of the device housing 100, thereby improving stability of connecting the blood component analysis assembly 300 to the device housing 100. Furthermore, a corresponding connector may be provided in the plug-in box 110, such that in a state where the blood component analysis assembly 300 is located in the plug-in box 110, the blood component analysis assembly 300 may be electrically connected to other members of the blood purification device (such as the power assembly or the main display screen 200), to implement communication connection or power supply connection there-between, etc.

In other specific embodiments, the connection part is located on an outer wall of the device housing 100, and the matching part is located on an outer wall of the blood component analysis assembly 300 and may be connected to the connection part. Here, the connection part may be a hook, and the matching part may be an opening or another hook cooperated with the hook, etc.

The blood component analysis assembly 300 is connected to the device housing 100 and the detection position 310 is exposed externally, therefore with adjustment of relative positions of the blood component analysis assembly 300 and the device housing 100, the device housing 100 may not shield the detection position 310, the blood sample may be placed at the detection position 310 from an outside of the device housing 100, and the blood sample may be acquired by an operator from the first blood pipeline, the second blood pipeline and/or the human blood vessel.

The blood sample is a blood sample in the first blood pipeline or a blood sample in the human blood vessel; or, the blood sample is a blood sample in the second blood pipeline. That is, the blood sample may be a first type of blood sample or a second type of blood sample. The first type of blood sample is a blood sample in the first blood pipeline or a blood sample in the human blood vessel; the second type of blood sample is a blood sample in the second blood pipeline.

The blood component analysis assembly 300 is provided with an accommodation structure, the accommodation structure corresponds to the detection position 310. The accommodation structure may be a plug-in slot, or a cavity with an on/off door, etc. The blood component analysis assembly 300 may be further configured to detect one or more of a calcium ion concentration, a sodium ion concentration, a potassium ion concentration, a chloride ion concentration and a magnesium ion concentration, in the blood sample; and/or, the blood component analysis assembly 300 may be further configured to detect one or more of a pH value, a pCO2 value, a pO2 value, a Glu value, a Lac value and a Hct value, of the blood sample. Furthermore, the blood purification device may further include an interaction member. Contents of the controller are the same as or similar to contents of the controller described in the previous blood purification device, which will not be described in detail here, and are all of which fall within the scope of protection of the application.

An embodiment of the application further provides a medical system, and the medical system includes a blood purification device and a blood gas analysis device. The blood purification device includes a device housing 100, a blood purifier, a blood pipeline assembly and a power assembly. The blood pipeline assembly includes a first blood pipeline connecting a human blood vessel to an inlet of the blood purifier, and a second blood pipeline connecting an outlet of the blood purifier to the human blood vessel. The power assembly includes at least one of a blood pump driving blood to flow in the blood pipeline assembly, a first replenishment pump enabling an anticoagulant solution to flow into the first blood pipeline, or a second replenishment pump enabling a calcium solution to flow into the second blood pipeline. The blood gas analysis device is configured to detect a blood sample, and the blood gas analysis device may be placed relatively independent of the blood purification device and communicatively connected to the blood purification device. The blood purification device and the blood gas analysis device may be placed relatively independent of each other, to improve flexibility of arrangement of the medical system.

The blood gas analysis device may have the same function as the above blood component analysis assembly 300, which will not be described in detail here.

Since the above blood purification device has the above technical effects, the medical system provided in the embodiment of the application has the same technical effects, which will not be described in detail here.

Same as above, the blood purification device further includes a controller. The blood gas analysis device is further configured to process a detection signal of the blood sample so as to obtain a detection result, and to output the detection result to the controller; or, the blood gas analysis device is further configured to output a detection signal of the blood sample to the controller, such that the controller processes the detection signal so as to obtain a detection result.

Furthermore, the controller is configured to adjust a flow rate of the first replenishment pump and/or the second replenishment pump according to the detection result; or, a flow rate of the first replenishment pump and/or the second replenishment pump is adjusted by an operator according to the detection result.

The controller may also provide an adjustment suggestion for the first replenishment pump and/or the second replenishment pump according to the detection result.

For convenience of usage, the blood purification device and the blood gas analysis device are arranged in a same medical room. With the above arrangement, it facilitates communication between the blood purification device and the blood gas analysis device. A separate medical room may be distinguished by a separate room. or may be distinguished by a corresponding area. For example, a region with a preset area in a large space (such as a floor area of 10*10 m²) is used as a medical room.

In consideration of simplifying devices and saving layout space, in the medical system provided in the embodiment of the application, there are multiple blood purification devices in number, and the blood gas analysis device is communicatively connected to the multiple blood purification devices. That is, the blood gas analysis device may be used as a common device for the multiple blood purification devices. Of course, there may also be multiple blood gas analysis devices in number, and the multiple blood gas analysis devices may correspond to the multiple blood purification devices one-to-one, and may be communicatively connected to the multiple blood purification devices.

Of course, it may also provide only one blood purification device in the medical system.

An embodiment of the application further provides a blood purification device, and the blood purification device includes a device housing 100, a blood purifier, a blood pipeline assembly, a power assembly, a controller and a blood component analysis assembly 300.

The blood component analysis assembly 300 is configured to detect an acquired blood sample, and the blood component analysis assembly is communicatively connected to the controller.

The blood purification device provided in the embodiment of the application may analyze the blood sample of the human body through the blood component analysis assembly 300, such that it is convenient to monitor a blood condition of the human body in time during corresponding operations performed by the blood purification device on the human body; with communicative connection between the blood component analysis assembly and the controller, it facilitates adjustment of the corresponding pump in the power assembly to make corresponding adjustments, and facilitates corresponding adjustments of the anticoagulant drugs and calcium ions in the blood purification circuit.

Furthermore, the blood component analysis assembly 300 is further configured to process a detection signal of the blood sample so as to obtain a detection result, and to output the detection result to the controller; or, the blood component analysis assembly 300 is further configured to output a detection signal of the blood sample to the controller, such that the controller processes the detection signal so as to obtain a detection result.

Modes of controlling other members in the blood purification device by the controller are the same as or similar to modes of the controller described in the above blood purification device, which will not be described in detail here. Furthermore, contents which may be detected by the blood component analysis assembly 300, sources of the blood sample and functions of the interaction member are the same as or similar to relevant contents in the above blood purification device, and descriptions thereof will not be repeated here.

In some other embodiments, a blood purification device includes a device housing 100, a blood purifier, a blood pipeline assembly, a power assembly and a blood component analysis assembly 300.

The device housing 100 may be configured to load some functional members of the blood purification device therein. The functional members may include a portion or all of the above members, i.e., the blood purifier, the blood pipeline assembly, the power assembly and the blood component analysis assembly 300.

Taking a CRRT device as an example, the blood purifier may include a filter configured to remove moisture and solutes from blood.

The blood pipeline assembly includes a first blood pipeline connecting a human blood vessel to an inlet of the blood purifier, and a second blood pipeline connecting an outlet of the blood purifier to the human blood vessel. Circulation of blood outside the human body may be achieved through the first blood pipeline and the second blood pipeline. The first blood pipeline is configured to transmit to-be-purified blood delivered by the human blood vessel to the blood purifier, and the second blood pipeline is configured to transmit purified blood delivered by the blood purifier to the human blood vessel. An anticoagulant solution may flow into the first blood pipeline, and a calcium solution may flow into the second blood pipeline. Whether the anticoagulant solution flows into the first blood pipeline, and a flow speed of the anticoagulant solution flowing into the first blood pipeline, may be controlled by a device for infusing the anticoagulant solution (relatively independent of the blood purification device). Furthermore, whether the calcium solution flows into the second blood pipeline, and a flow speed of the calcium solution flowing into the second blood pipeline, may be controlled by a device for infusing the calcium solution (relatively independent of the blood purification device). The anticoagulant solution is enabled to flow into the first blood pipeline, such that the anticoagulant solution acts on blood in the first blood pipeline, to avoid occurrence of blood coagulation after the blood flows out of the human blood vessel and comes into contact with pipelines of the blood purification device and a filtration structure (such as a dialysis membrane or other structures) of the blood purifier, to ensure an effective purification operation of the blood. The calcium solution is enabled to flow into the second blood pipeline, such that the calcium solution acts on blood in the second blood pipeline, to avoid the human body from suffering from hypocalcemia or physical discomfort due to the calcium ion concentration in the blood not falling within a standard range, after the blood flows into the human blood vessel. A first device for infusing the anticoagulant solution and a second device for infusing the calcium solution may be detachably arranged on the blood purification device, or may be arranged separately from the blood purification device.

The blood component analysis assembly 300 is configured to detect an acquired blood sample. The blood component analysis assembly 300 may be arranged in the device housing 100, and the blood component analysis assembly is configured to detect an acquired blood sample. An acquisition operation of the blood sample of the human body is implemented by manual sampling or an automatic acquisition device, and the blood sample is placed at a detection position 310 by a manual or mechanical operation, such that the blood component analysis assembly 300 may detect the blood sample. Furthermore, the device housing 100 may be provided with a connection part, and the blood component analysis assembly 300 may be provided with a matching part connected to the connection part. The blood component analysis assembly is configured to detect an acquired blood sample.

The blood purification device provided in the embodiment of the application may analyze the blood sample of the human body through the blood component analysis assembly 300, such that it is convenient to monitor a blood condition of the human body in time during corresponding operations performed by the blood purification device on the human body, to facilitate adjustment of the corresponding pump in the power assembly to make corresponding adjustments, and facilitate corresponding adjustments of the anticoagulant drugs and calcium ions in the blood purification circuit.

Specifically, the blood component analysis assembly 300 may be configured to detect one or more of a calcium ion concentration, a sodium ion concentration, a potassium ion concentration, a chloride ion concentration and a magnesium ion concentration, in the blood sample. Taking the sodium ion concentration as an example, during treatment of the patient with the blood purification device, for the patient suffering from hypernatremia or hyponatremia, the sodium ion concentration in the blood sample is detected by the blood component analysis assembly 300, to facilitate monitoring the sodium ion concentration in the blood, and facilitate finding and correcting the above various diseases in time. Similarly, during treatment of the patient with the blood purification device, for the patient suffering from hyperkalemia, hypokalemia or other abnormal electrolyte levels, it also needs to monitor corresponding electrolyte concentrations in the blood at a regular time.

In a process of monitoring the above ion concentrations, the blood purification device may also provide a warning reminder according to the detection result, to remind the doctor/nurse to find the patient's symptom in time and make corresponding processing. For example, the blood purification device may provide a reminder message such as "Sodium ion concentration is too high, please process it!", etc. Or, concentrations of various ions monitored at different time points may be presented in a form of trend graph, to facilitate the doctor/nurse to observe and review status of the patient throughout the treatment process.

Furthermore, the blood component analysis assembly 300 may also be configured to detect one or more of a pH (hydrogen ion concentration) value, a pCO2 (Partial Pressure of Carbon Dioxide) value, a pO2 (partial pressure of oxygen) value, a Glu (glucose) value, a Lac (Lactate) value and a Hct (Hematocrit) value, of the blood sample. Through corresponding detection operations, it facilitates the operator to perform corresponding processing according to the detection result.

As shown in the above embodiment, the power assembly of the blood purification device further includes a first replenishment pump enabling an anticoagulant solution to flow into the first blood pipeline, and/or a second replenishment pump enabling a calcium solution to flow into the second blood pipeline. The first replenishment pump may control the anticoagulant solution flowing into the first blood pipeline, including that the first replenishment pump controls whether the anticoagulant solution flows into the first blood pipeline, and controls a flow speed of the anticoagulant solution flowing into the first blood pipeline. The anticoagulant solution is enabled to flow into the first blood pipeline by the first replenishment pump, such that the anticoagulant solution acts on blood in the first blood pipeline, to avoid occurrence of blood coagulation after the blood flows out of the human blood vessel and comes into contact with pipelines of the blood purification device and a filtration structure (such as a dialysis membrane or other structures) of the blood purifier, to ensure an effective purification operation of the blood. The second replenishment pump may control the calcium solution flowing into the second blood pipeline, including that the second replenishment pump controls whether the calcium solution flows into the second blood pipeline, and controls a flow speed of the calcium solution flowing into the second blood pipeline. The calcium solution is enabled to flow into the second blood pipeline by the second replenishment pump, such that the calcium solution acts on blood in the second blood pipeline, to avoid the human body from suffering from hypocalcemia or physical discomfort due to the calcium ion concentration in the blood not falling within a standard range, after the blood flows into the human blood vessel.

Or, the anticoagulant solution may be enabled to flow into the first blood pipeline by an external power device, and/or the calcium solution may be enabled to flow into the second blood pipeline by an external power device. It may be understood that the external power device may be a device which is relatively independent of the blood purification device provided in the embodiment of the application, and the external power device may be connected to the blood purification device by wireless or wired communication; or, the external power device may be relatively independent of the blood purification device completely, and there is no connection structure there-between.

The external power device includes a first replenishment pump enabling the anticoagulant solution to flow into the first blood pipeline, and/or a second replenishment pump enabling the calcium solution to flow into the second blood pipeline. That is, the first replenishment pump and/or the second replenishment pump do not belong to the blood purification device, and the first replenishment pump and/or the second replenishment pump are independently located outside the blood purification device. The first replenishment pump and/or the second replenishment pump may be controlled separately, or the blood purification device may be communicatively connected to the first replenishment pump and/or the second replenishment pump.

That is, the anticoagulant solution may flow into the first blood pipeline, which may be implemented by the first replenishment pump included in the power assembly of the blood purification device or by the first replenishment pump included in the external power device; the calcium solution may flow into the second blood pipeline, which may be implemented by the second replenishment pump included in the power assembly of the blood purification device or by the second replenishment pump included in the external power device.

An end of an output pipeline of the first replenishment pump may be connected to an anticoagulant solution bag, and another end of the output pipeline of the first replenishment pump may be connected to an interface on the first blood pipeline, to implement that the anticoagulant solution flows into the first blood circuit; an end of an output pipeline of the second replenishment pump may be connected to a calcium solution bag, and another end of the output pipeline of the second replenishment pump may be connected to an interface on the second blood pipeline, to implement that the calcium solution flows into the second blood circuit.

In order to facilitate controlling flow rates, in some embodiments, the blood component analysis assembly may be configured to process a detection signal of the blood sample to obtain a detection result, and to output the detection result to a controller of the blood purification device. That is, the blood component analysis assembly 300 processes a detection signal of the blood sample to obtain a detection result, and outputs the detection result to the controller. That is, after the blood component analysis assembly 300 detects the blood sample and obtains a detection signal, the blood component analysis assembly 300 further processes the detection signal to obtain the detection result (such as a specific concentration value, etc.), the detection result is transmitted by the blood component analysis assembly 300 to the controller.

Or, the blood component analysis assembly is configured to output a detection signal of the blood sample to the controller of the blood purification device, such that the controller processes the detection signal so as to obtain a detection result. That is, after the blood component analysis assembly 300 detects the blood sample and obtains a detection signal, the blood component analysis assembly 300 transmits the detection signal to the controller, and the controller processes the detection signal to obtain the detection result (such as a specific concentration value, etc.). There is a corresponding logic operation to obtain the detection result from the detection signal, and in the embodiment, the logic operation is implemented by the controller. The controller may be a main control board in the blood purification device, and a unit configured to implement the above logic operation may be integrated into the main control board.

The controller may be configured to send a control signal for adjusting a flow rate of the anticoagulant solution flowing into the first blood pipeline and/or a control signal for adjusting a flow rate of the calcium solution flowing into the second blood pipeline, according to the detection result; or, the controller may be configured to provide an adjustment suggestion for the flow rate of the anticoagulant solution flowing into the first blood pipeline and/or the flow rate of the calcium solution flowing into the second blood pipeline, according to the detection result.

That is, the controller may perform corresponding processing on the blood purification device according to the detection result. The corresponding processing may include control and adjustments of the flow rate of the anticoagulant solution and the flow rate of the calcium solution, or may provide adjustment suggestions thereof.

Taking an example that the above external power device includes a first replenishment pump enabling the anticoagulant solution to flow into the first blood pipeline, and/or a second replenishment pump enabling the calcium solution to flow into the second blood pipeline, then the corresponding processing may include control and adjustments of the flow rate of the anticoagulant solution and the flow rate of the calcium solution.

The controller sends a control signal for adjusting the flow rate of the anticoagulant solution flowing into the first blood pipeline, which may control adjustment and start-stop of the first replenishment pump or the like, to avoid occurrence of adverse effects due to concentrations of component substances (including the anticoagulant solution) in the blood failing to reach their standard values. The controller sends a control signal for adjusting the flow rate of the calcium solution flowing into the second blood pipeline, which may control adjustment and start-stop of the second replenishment pump or the like, to avoid occurrence of adverse effects due to concentrations of component substances (including the calcium solution) in the blood failing to reach their standard values.

The above control signal of the flow rate may also be transmitted to other devices, to facilitate the operator to obtain the control signal, and then the operator may control the first replenishment pump and/or the second replenishment pump correspondingly.

Furthermore, the adjustment suggestion for the flow rate of the anticoagulant solution flowing into the first blood pipeline and/or the flow rate of the calcium solution flowing into the second blood pipeline provided by the controller according to the detection result, may be obtained by the operator (for example, the adjustment suggestion is displayed on a display screen, or broadcasted by a voice device, etc.), and then the operator determines whether the flow rate of the anticoagulant solution flowing into the first blood pipeline and/or the flow rate of the calcium solution flowing into the second blood pipeline are adjusted.

Taking an example that the above external power device includes a first replenishment pump enabling the anticoagulant solution to flow into the first blood pipeline and/or a second replenishment pump enabling the calcium solution to flow into the second blood pipeline, then when the operator determines whether the flow rate of the anticoagulant solution flowing into the first blood pipeline and/or the flow rate of the calcium solution flowing into the second blood pipeline are adjusted, it may be whether the first replenishment pump and/or the second replenishment pump are adjusted correspondingly.

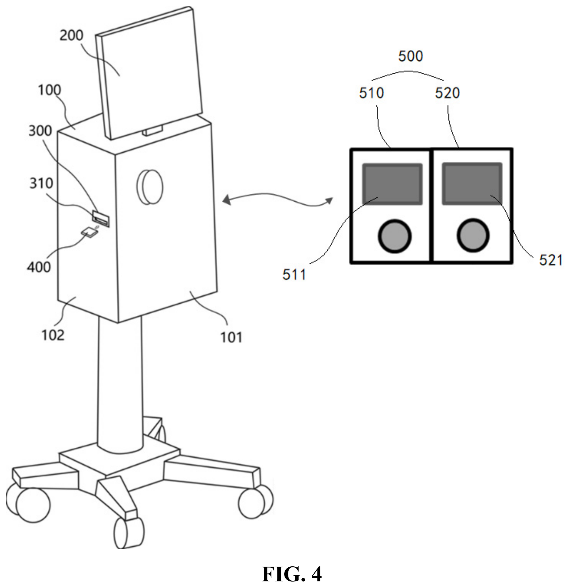

As shown in FIG. 4, an embodiment of the application further provides a medical system, and the medical system includes a blood purification device as shown above, and further includes an auxiliary power device 500. The auxiliary power device 500 includes a first replenishment pump 510 enabling an anticoagulant solution to flow into the first blood pipeline, and/or a second replenishment pump 520 enabling a calcium solution to flow into the second blood pipeline. The first replenishment pump 510 and/or the second replenishment pump 520 are power members which are independently located outside the blood purification device.

In some embodiments, the auxiliary power device 500 is communicatively connected to the blood purification device, including wired connection, wireless connection, etc. The blood purification device may be communicatively connected to the first replenishment pump 510 and/or the second replenishment pump 520 in the auxiliary power device 500, such that start-stop of the first replenishment pump 510 and/or the second replenishment pump 520 and flow rates infused by the first replenishment pump 510 and/or the second replenishment pump 520 may be controlled according to the blood purification device.

The blood purification device may further include an interaction member, the interaction member includes a display screen, the display screen is configured to display a delivery flow speed of the first replenishment pump 510 and/or the second replenishment pump 520.

Or, the blood component analysis assembly may be configured to process a detection signal of the blood sample to obtain a detection result, and to output the detection result to a controller of the blood purification device; or, the blood component analysis assembly is configured to output a detection signal of the blood sample to the controller of the blood purification device, such that the controller processes the detection signal so as to obtain a detection result. The controller is configured to adjust a flow rate of the first replenishment pump 510 and/or the second replenishment pump 520 according to the detection result; or, the controller is configured to provide an adjustment suggestion for the first replenishment pump 510 and/or the second replenishment pump 520 according to the detection result.

In some other embodiments, the auxiliary power device 500 is relatively independent of the blood purification device. That is, the auxiliary power device 500 and the blood purification device may be placed relatively independent of each other, to improve flexibility of arrangement of the medical system.

The blood component analysis assembly may be configured to process a detection signal of the blood sample to obtain a detection result, and to output the detection result to a controller of the blood purification device; or, the blood component analysis assembly is configured to output a detection signal of the blood sample to the controller of the blood purification device, such that the controller processes the detection signal so as to obtain a detection result.

Furthermore, a flow rate of the first replenishment pump 510 and/or the second replenishment pump 520 is adjusted by an operator according to the detection result. The detection result may include a specific concentration value or the like, or may include an indication that a corresponding component is relatively high or low (for example, an information indication such as "a (venous) free calcium concentration after the filter: 0.45 mmol/L (relatively high)", etc.) or the like, to facilitate the operator to manually adjust the flow rate of the first replenishment pump 510 and/or the second replenishment pump 520 according to the detection result. The auxiliary power device 500 may be provided with a display structure, the display structure is configured to display a delivery flow speed of the first replenishment pump 510 and/or the second replenishment pump 520. The display structure may include a first display 511 of the first replenishment pump 510, and the first display 511 may display the delivery flow speed of the first replenishment pump 510, such that the operator may adjust the delivery flow speed of the first replenishment pump 510 correspondingly according to displayed parameters. The display structure may further include a second display 521 of the second replenishment pump 520, and the second display 521 may display the delivery flow speed of the second replenishment pump 520, such that the operator may adjust the delivery flow speed of the second replenishment pump 520 correspondingly according to displayed parameters.

In other embodiments, a blood purification device includes a device housing 100, a blood purifier, a blood pipeline assembly, a power assembly and an electrolyte component analysis assembly.

Similarly, the device housing 100 may be configured to load some functional members of the blood purification device therein. The functional members may include a portion or all of the above members, i.e., the blood purifier, the blood pipeline assembly, the power assembly and the electrolyte component analysis assembly.

Taking a CRRT device as an example, the blood purifier may include a filter configured to remove moisture and solutes from blood.

The blood pipeline assembly includes a first blood pipeline connecting a human blood vessel to an inlet of the blood purifier, and a second blood pipeline connecting an outlet of the blood purifier to the human blood vessel. Circulation of blood outside the human body may be achieved through the first blood pipeline and the second blood pipeline. The first blood pipeline is configured to transmit to-be-purified blood delivered by the human blood vessel to the blood purifier, and the second blood pipeline is configured to transmit purified blood delivered by the blood purifier to the human blood vessel. An anticoagulant solution may flow into the first blood pipeline, and a calcium solution may flow into the second blood pipeline. Whether the anticoagulant solution flows into the first blood pipeline, and a flow speed of the anticoagulant solution flowing into the first blood pipeline, may be controlled by a device for infusing the anticoagulant solution (relatively independent of the blood purification device). Furthermore, whether the calcium solution flows into the second blood pipeline, and a flow speed of the calcium solution flowing into the second blood pipeline, may be controlled by a device for infusing the calcium solution (relatively independent of the blood purification device). The anticoagulant solution is enabled to flow into the first blood pipeline, such that the anticoagulant solution acts on blood in the first blood pipeline, to avoid occurrence of blood coagulation after the blood flows out of the human blood vessel and comes into contact with pipelines of the blood purification device and a filtration structure (such as a dialysis membrane or other structures) of the blood purifier, to ensure an effective purification operation of the blood. The calcium solution is enabled to flow into the second blood pipeline, such that the calcium solution acts on blood in the second blood pipeline, to avoid the human body from suffering from hypocalcemia or physical discomfort due to the calcium ion concentration in the blood not falling within a standard range, after the blood flows into the human blood vessel. A first device for infusing the anticoagulant solution and a second device for infusing the calcium solution may be detachably arranged on the blood purification device, or may be arranged separately from the blood purification device.

The electrolyte component analysis assembly is configured to detect an acquired waste liquid sample. The electrolyte component analysis assembly may be arranged in the device housing 100, and the electrolyte component analysis assembly is configured to detect an acquired waste liquid sample. An acquisition operation of a waste liquid sample generated by the blood purification device due to a blood purification operation (such as treatment by blood purification) is implemented by manual sampling or an automatic acquisition device, and the waste liquid sample is placed at a corresponding detection position (such as a detection position 310) by a manual or mechanical operation, such that the electrolyte component analysis assembly may detect the waste liquid sample. Furthermore, the device housing 100 may be provided with a connection part, and the electrolyte component analysis assembly may be provided with a matching part connected to the connection part. The electrolyte component analysis assembly is configured to detect an acquired waste liquid sample.

The blood purification device further includes a sampling member, the sampling member may acquire the waste liquid sample from a waste liquid pipeline or a waste liquid collection device of the blood purification device.

The blood purification device provided in the embodiment of the application may analyze a waste liquid sample generated by a purification operation of acquired blood through the electrolyte component analysis assembly, such that it is convenient to monitor an electrolyte concentration condition of the human body in time during corresponding operations performed by the blood purification device on the human body, to facilitate adjustment of the corresponding pump in the power assembly to make corresponding adjustments, and facilitate corresponding adjustments of the calcium ions in the blood purification circuit.

Specifically, the electrolyte component analysis assembly may be configured to detect one or more of a calcium ion concentration, a sodium ion concentration, a potassium ion concentration, a chloride ion concentration and a magnesium ion concentration, in the waste liquid sample. Taking the sodium ion concentration as an example, during treatment of the patient with the blood purification device, for the patient suffering from hypernatremia or hyponatremia, the sodium ion concentration in the waste liquid sample is detected by the electrolyte component analysis assembly, to facilitate monitoring the sodium ion concentration in the blood, and facilitate finding and correcting the above various diseases in time. Similarly, during treatment of the patient with the blood purification device, for the patient suffering from hyperkalemia, hypokalemia or other abnormal electrolyte levels, it also needs to monitor corresponding electrolyte concentrations in the blood at a regular time.

In a process of monitoring the above ion concentrations, the blood purification device may also provide a warning reminder according to the detection result, to remind the doctor/nurse to find the patient's symptom in time and make corresponding processing. For example, the blood purification device may provide a reminder message such as "Sodium ion concentration is too high, please process it!", etc. Or, concentrations of various ions monitored at different time points may be presented in a form of trend graph, to facilitate the doctor/nurse to observe and review status of the patient throughout the treatment process.

As shown in the above embodiment, the power assembly of the blood purification device further includes a first replenishment pump enabling the anticoagulant solution to flow into the first blood pipeline, and/or a second replenishment pump enabling the calcium solution to flow into the second blood pipeline. The first replenishment pump may control the anticoagulant solution flowing into the first blood pipeline, including that the first replenishment pump controls whether the anticoagulant solution flows into the first blood pipeline, and controls a flow speed of the anticoagulant solution flowing into the first blood pipeline. The anticoagulant solution is enabled to flow into the first blood pipeline by the first replenishment pump, such that the anticoagulant solution acts on blood in the first blood pipeline, to avoid occurrence of blood coagulation after the blood flows out of the human blood vessel and comes into contact with pipelines of the blood purification device and a filtration structure (such as a dialysis membrane or other structures) of the blood purifier, to ensure an effective purification operation of the blood. The second replenishment pump may control the calcium solution flowing into the second blood pipeline, including that the second replenishment pump controls whether the calcium solution flows into the second blood pipeline, and controls a flow speed of the calcium solution flowing into the second blood pipeline. The calcium solution is enabled to flow into the second blood pipeline by the second replenishment pump, such that the calcium solution acts on blood in the second blood pipeline, to avoid the human body from suffering from hypocalcemia or physical discomfort due to the calcium ion concentration in the blood not falling within a standard range, after the blood flows into the human blood vessel.

Or, the anticoagulant solution may be enabled to flow into the first blood pipeline by an external power device, and/or the calcium solution may be enabled to flow into the second blood pipeline by an external power device. Here, the external power device enabling the anticoagulant solution to flow into the first blood pipeline and the external power device enabling the calcium solution to flow into the second blood pipeline may be a same external power device, or may be different external power devices. It may be understood that the external power device may be a device which is relatively independent of the blood purification device provided in the embodiment of the application, and the external power device may be connected to the blood purification device by wireless or wired communication; or, the external power device may be relatively independent of the blood purification device completely, and there is no connection structure there-between.

Furthermore, the electrolyte composition analysis assembly may be further configured to detect an acquired blood sample. That is, the electrolyte component analysis assembly may have functions of the blood component analysis assembly 300 in the above embodiment. Therefore, it may also perform corresponding control as described in the above embodiment on the blood purification device, by combining a detection result of detecting the waste liquid sample (that is, a first detection result) with a detection result of detecting the blood sample (that is, a second detection result). Of course, a blood component analysis assembly 300 may also be additionally provided, the blood component analysis assembly 300 is relatively independent of the electrolyte component analysis assembly, and the blood component analysis assembly 300 is configured to detect the acquired blood sample, as shown in the above embodiment.

An example that the blood purification device is controlled correspondingly by the detection result of detecting the waste liquid sample is taken. In order to facilitate controlling flow rates, in some embodiments, the electrolyte component analysis assembly may be configured to process a detection signal of the waste liquid sample to obtain a detection result, and to output the detection result to a controller of the blood purification device. That is, the electrolyte component analysis assembly processes the detection signal of the waste liquid sample to obtain a detection result, and outputs the detection result to the controller. That is, after the electrolyte component analysis assembly detects the waste liquid sample and obtains a detection signal, the electrolyte component analysis assembly further processes the detection signal to obtain the detection result (such as a specific concentration value, etc.), the detection result is transmitted by the electrolyte component analysis assembly to the controller.

Or, the electrolyte component analysis assembly is configured to output a detection signal of the waste liquid sample to the controller of the blood purification device, such that the controller processes the detection signal so as to obtain a detection result. That is, after the electrolyte component analysis assembly detects the waste liquid sample and obtains a detection signal, the electrolyte component analysis assembly transmits the detection signal to the controller, and the controller processes the detection signal to obtain the detection result (such as a specific concentration value, etc.). There is a corresponding logic operation to obtain the detection result from the detection signal, and in the embodiment, the logic operation is implemented by the controller. The controller may be a main control board in the blood purification device, and a unit configured to implement the above logic operation may be integrated into the main control board.

The controller may be configured to send a control signal for adjusting a flow rate of the calcium solution flowing into the second blood pipeline, according to the detection result; or, the controller is configured to provide an adjustment suggestion for the flow rate of the calcium solution flowing into the second blood pipeline, according to the detection result.

That is, the controller may perform corresponding processing on the blood purification device according to the detection result. The corresponding processing may include control and adjustment of the flow rate of the calcium solution, or may provide adjustment suggestion thereof.

Taking an example that the above external power device includes a first replenishment pump enabling the anticoagulant solution to flow into the first blood pipeline, and/or a second replenishment pump enabling the calcium solution to flow into the second blood pipeline, then the corresponding processing may include control and adjustments of the flow rate of the anticoagulant solution and the flow rate of the calcium solution.

Control and adjustment of the flow rate of the anticoagulant solution may be performed according to the detection result of detecting the blood sample (by using the electrolyte component analysis assembly or the additionally provided blood component analysis assembly 300), which is specifically as shown in the above embodiment and is not specifically limited here.

Control and adjustment of the flow rate of the calcium solution may be that the controller sends a control signal for adjusting the flow rate of the calcium solution flowing into the second blood pipeline, which may control adjustment and start-stop of the second replenishment pump or the like, to avoid occurrence of adverse effects due to concentrations of component substances (including the calcium solution) in the blood failing to reach their standard values.

The above control signal of the flow rate may also be transmitted to other devices, to facilitate the operator to obtain the control signal, and then the operator may control the second replenishment pump correspondingly.

Furthermore, the adjustment suggestion for the flow rate of the calcium solution flowing into the second blood pipeline provided by the controller according to the detection result, may be obtained by the operator (for example, the adjustment suggestion is displayed on a display screen, or broadcasted by a voice device, etc.), and then the operator determines whether the flow rate of the calcium solution flowing into the second blood pipeline is adjusted.

Taking an example that the above external power device includes a second replenishment pump enabling the calcium solution to flow into the second blood pipeline, then when the operator determines whether the flow rate of the calcium solution flowing into the second blood pipeline is adjusted, it may be whether the second replenishment pump is adjusted correspondingly.

Various embodiments in the description are described in a progressive manner, each embodiment focus on describing differences between it and other embodiments, and the same and similar portions between various embodiments may refer to each other.

The foregoing descriptions of disclosed embodiments enable those skilled in the art to implement or use the application. Various modifications to these embodiments will be apparent to those skilled in the art, and general principles defined here may be implemented in other embodiments without departing from the spirit or scope of the application. Therefore, the application will not be limited to these embodiments shown here, instead, should be conform to a broadest scope consistent with principles and novel characteristics disclosed here.

## Claims

1. A blood purification device, comprising:
a device housing (100);
a blood purifier;
a blood pipeline assembly, comprising a first blood pipeline connecting a human blood vessel to an inlet of the blood purifier, and a second blood pipeline connecting an outlet of the blood purifier to the human blood vessel;
a power assembly, comprising at least one of: a blood pump driving blood to flow in the blood pipeline assembly, a first replenishment pump enabling an anticoagulant solution to flow into the first blood pipeline, and a second replenishment pump enabling a calcium solution to flow into the second blood pipeline; and
a blood component analysis assembly (300), arranged in the device housing (100), and configured to detect an acquired blood sample.

2. A blood purification device, comprising:
a device housing (100), provided with a connection part (110);
a blood purifier;
a blood pipeline assembly, comprising a first blood pipeline connecting a human blood vessel to an inlet of the blood purifier, and a second blood pipeline connecting an outlet of the blood purifier to the human blood vessel;
a power assembly, comprising at least one of a blood pump driving blood to flow in the blood pipeline assembly, a first replenishment pump enabling an anticoagulant solution to flow into the first blood pipeline, and a second replenishment pump enabling a calcium solution to flow into the second blood pipeline; and
a blood component analysis assembly (300), provided with a matching part connected to the connection part, and configured to detect an acquired blood sample.

3. A blood purification device, comprising:
a device housing (100);
a blood purifier;
a blood pipeline assembly, comprising a first blood pipeline connecting a human blood vessel to an inlet of the blood purifier, and a second blood pipeline connecting an outlet of the blood purifier to the human blood vessel, wherein an anticoagulant solution is capable of flowing into the first blood pipeline, and a calcium solution is capable of flowing into the second blood pipeline;
a power assembly, comprising a blood pump driving blood to flow in the blood pipeline assembly; and
a blood component analysis assembly (300), arranged in the device housing (100), and configured to detect an acquired blood sample.

4. A blood purification device, comprising:
a device housing (100), provided with a connection part;
a blood purifier;
a blood pipeline assembly, comprising a first blood pipeline connecting a human blood vessel to an inlet of the blood purifier, and a second blood pipeline connecting an outlet of the blood purifier to the human blood vessel, wherein an anticoagulant solution is capable of flowing into the first blood pipeline, and a calcium solution is capable of flowing into the second blood pipeline;
a power assembly, comprising a blood pump driving blood to flow in the blood pipeline assembly; and
a blood component analysis assembly (300), provided with a matching part connected to the connection part, and configured to detect an acquired blood sample.

5. The blood purification device of any of claims 1 to 4, further comprising a controller,
wherein the blood component analysis assembly (300) is further configured to process a detection signal of the blood sample so as to obtain a detection result, and to output the detection result to the controller; or
the blood component analysis assembly (300) is further configured to output a detection signal of the blood sample to the controller, such that the controller processes the detection signal so as to obtain a detection result.

6. The blood purification device of claim 5, wherein the anticoagulant solution is a citric acid solution, and/or the detection result comprises at least a calcium ion concentration in the blood sample.

7. The blood purification device of claim 1 or 3, wherein the blood component analysis assembly (300) has a detection position (310), the blood sample is capable of being placed at the detection position (310) from an outside of the device housing (100);
or,
wherein the blood component analysis assembly (300) has a detection position (310), the blood sample is capable of being placed at the detection position (310) from an outside of the device housing (100), and the device housing (100) is provided with an accommodation structure corresponding to the detection position, a test card (400) carrying the blood sample, which card is capable of being placed in the accommodation structure, to enable the blood sample to be placed at the detection position.

8. The blood purification device of claim 5, wherein the controller is configured to adjust a flow rate of the first replenishment pump and/or the second replenishment pump according to the detection result;
or,
a flow rate of the first replenishment pump and/or the second replenishment pump is adjusted by an operator according to the detection result.

9. The blood purification device of claim 5, wherein the controller is configured to provide an adjustment suggestion for the first replenishment pump and/or the second replenishment pump according to the detection result.

10. The blood purification device of any of claims 1 to 9, wherein the blood component analysis assembly (300) is further configured to detect one or more of a calcium ion concentration, a sodium ion concentration, a potassium ion concentration, a chloride ion concentration and a magnesium ion concentration, in the blood sample; and/or
the blood component analysis assembly (300) is further configured to detect one or more of a pH value, a pCO2 value, a pO2 value, a Glu value, a Lac value and a Het value, of the blood sample.

11. The blood purification device of any one of claims 1 to 10, further comprising: an interaction member (200), communicatively connected to the blood component analysis assembly (300), and configured to output a detection result of at least one ion concentration.

12. The blood purification device of claim 2 or 4, wherein the connection part (110) is detachably connected to the matching part.

13. The blood purification device of claim 2 or 4, wherein the connection part (110) is a plug-in box with an opening located on an outer wall of the device housing (100) and recessed toward an interior of the device housing (100), and the matching part is a housing wall of the blood component analysis assembly (300); and the blood component analysis assembly (300) is pluggable into the plug-in box;
or,
wherein the connection part (110) is located on an outer wall of the device housing (100), and the matching part is located on an outer wall of the blood component analysis assembly (300) and is connectable to the connection part.

14. The blood purification device of claim 3 or 4, wherein the anticoagulant solution is enabled to flow into the first blood pipeline by an external power device, and/or the calcium solution is enabled to flow into the second blood pipeline by an external power device.

15. The blood purification device of claim 3 or 4, wherein the blood component analysis assembly (300) is further configured to process a detection signal of the blood sample so as to obtain a detection result, and to output the detection result to a controller of the blood purification device; or, the blood component analysis assembly (300) is further configured to output a detection signal of the blood sample to the controller of the blood purification device, such that the controller processes the detection signal to obtain a detection result;
the controller is configured to send a control signal for adjusting a flow rate of the anticoagulant solution flowing into the first blood pipeline and/or a control signal for adjusting a flow rate of the calcium solution flowing into the second blood pipeline, according to the detection result; or, the controller is configured to provide an adjustment suggestion for a flow rate of the anticoagulant solution flowing into the first blood pipeline and/or a flow rate of the calcium solution flowing into the second blood pipeline, according to the detection result.
